Europäisches Patentamt

European Patent Office

Office européen des brevets

(19)

(11) **EP 0 616 231 B1**

(12) **EUROPÄISCHE PATENTSCHRIFT**

(45) Veröffentlichungstag und Bekanntmachung des Hinweises auf die Patenterteilung:
**04.03.1998 Patentblatt 1998/10**

(51) Int Cl.⁶: **G01S 7/52**, G01S 15/89, A61B 8/06

(21) Anmeldenummer: **94109331.2**

(22) Anmeldetag: **19.10.1989**

(54) **Verfahren und Messanordnung zum Messen des Volumenstromes in einer Schicht mit reflektierender Struktur**

Process and device for measuring the current volume in a layer with reflecting structure

Procédé et dispositif pour mesurer le volume de courant dans une couche à structure réfléchissante

(84) Benannte Vertragsstaaten:
**AT BE CH DE FR GB IT LI LU NL SE**

(30) Priorität: **19.10.1988 CH 3886/88**

(43) Veröffentlichungstag der Anmeldung:
**21.09.1994 Patentblatt 1994/38**

(62) Dokumentnummer(n) der früheren Anmeldung(en) nach Art. 76 EPÜ:
**89912415.0 / 0 394 428**

(73) Patentinhaber: **Institut für Biomedizinische Technik und Medizinische Informatik der Universität und ETH Zürich
8044 Zürich (CH)**

(72) Erfinder: **Moser, Urs
Ch-8006 Zürich (CH)**

(74) Vertreter: **Troesch Scheidegger Werner AG Patentanwälte,
Siewerdtstrasse 95,
Postfach
8050 Zürich (CH)**

(56) Entgegenhaltungen:
EP-A- 0 139 285          DE-A- 3 605 163
US-A- 4 596 145          US-A- 4 608 868
US-A- 4 800 891

• ULTRASONIC IMAGING, Bd.8, Nr.2, April 1986, DULUTH, MN, US Seiten 73 - 85 O. BONNEFOUS ET AL. 'Time Domain FOrmulation of Pulse-doppler Ultrasound and Blood Velocity Estimation by Cross Correlation'

**Beschreibung**

Die vorliegende Erfindung betrifft ein Verfahren und eine Messanordnung nach dem Oberbegriff von Anspruch 1 bzw. nach dem Oberbegriff von Anspruch 14.

Insbesondere im Zusammenhang mit der Ultraschall-Puls-Dopplertechnik zur Messung von Strömungsgeschwindigkeiten in einem Streuzentren mitführenden Fluidum ist es seit über zwei Jahrzehnten bekannt (Baker, "Pulsed Ultrasonic Blood Flow Sensing", IEEE Trans. SU-17/3, p. 170 (1970)), durch Auswerten der Doppler-Frequenz an einem aus einer bestimmten Distanz stammenden Echosignal bzw. dessen Phasenlage bezüglich eines stabilen Referenzsignals, die Geschwindigkeit des Fluidums zu bestimmen. Dabei wird aufgrund des Dopplereffektes nur die in Strahlrichtung zeigende Geschwindigkeitskomponente ermittelt.

Aufgrund der an einer zweidimensionalen Schicht bezüglich eines Empfängers bestehenden unterschiedlichen Distanzen konnte, nach der im obgenannten Artikel bekannten Technik, eine zweidimensionale Struktur nicht gemessen werden, auch bei Anwendung des erwähnten, bekannten Verfahrens auf eine stationäre.

In einem weiter entwickelten, sog. vielkanaligen Gerät (McLeod, "A Multiple Gate Pulsed Doppler Flowmeter", Proc. IEEE Ultrasonic Symp. Miami, (1970)) wurden, sequentiell, die Echos ausschliesslich aus verschiedenen Distanzen ausgewertet und quasi simultan die Resultate angezeigt, entsprechend einem Geschwindigkeitsprofil entlang des Beschallungsstrahls. Auch bei diesem Verfahren ist aufgrund obgenannter Distanzverhältnisse an einer zweidimensionalen Schicht und des eigentlich eindimensionalen Messprinzips, eine Messung eingangs genannter Art nicht möglich.

Aus der DE-PS 24 06 630 wurde im weiteren ein Verfahren zur Messung eines Geschwindigkeitsprofils eines reflektierende Strukturen mitführenden, fliessenden Fluides, aus der Auswertung von Schall- oder Ultraschallechos bekannt, durch welches es gelang, das Geschwindigkeitsprofil ohne eigentliche Distanzvorgabe zu ermitteln. Auch mit diesem Verfahren ist es aber nicht möglich, aufgrund obgenannter Distanzverhältnisse eine zweidimensional ausgedehnte Schicht bezüglich reflektierender Strukturen auszumessen, im Falle, dass das genannte Vorgehen auf stationäre Strukturen angewandt wird.

Im weiteren ist es bekannt, mittels sog. Phased-Array-Sektorscannern, den Schallstrahl in einer die Sender/Ziel-Achse enthaltenen Ebene, durch unterschiedliche Senderanregung, unterschiedlich auszulenken und so Streuzentrengeschwindigkeiten jeweils entlang der momentan angesteuerten Beschallungsachse, sequentiell in verschiedenen Richtungen in besagter Ebene zu ermitteln, so dass sequentiell in dieser Schnittebene, in dem dem Fachmann bekannten "B-Modus" das zweidimensionale Geschwindigkeitsfeld abgetastet wird.

Bei diesem Vorgehen ist, auch bei stationären Strukturen, eine Echografie nur einer Schnittfläche, welche in der genannten Ablenkebene liegt, möglich. Bei Anwendung des genannten Verfahrens für seinen eigentlichen Zweck, nämlich die Messung eines Geschwindigkeitsprofils, entsteht ein weiteres Problem: Für eine Echtzeitmessung muss die Messzeit für das Abtasten in der Ebene limitiert werden, so dass entweder pro momentane Strahlrichtung nur kurze Messzeitspannen zur Verfügung stehen oder aber, bei Verlängerung der genannten Zeitspannen, nur einige wenige Abtastrichtungen ausgemessen werden können.

Bei den sog. Color-Doppler-Geräten (Chihiro, "Real-Time Two-Dimensional Blood Flow Imaging Using an Autocorrelation Technique", IEEE Trans. on Sonics and Ultrasonics, Vol, SU 32, No. 3, (1985)) wurde vor allem die Messzeit in eine jeweilige Strahlrichtung stark limitiert, so dass sich, aufgrund der Unschärferelation, die Dopplerfrequenz nur sehr ungenau bestimmen lässt.

Die vorliegende Erfindung setzt sich zum Ziel, ein Verfahren eingangs genannter Art bzw. eine entsprechende Vorrichtung zu schaffen, mittels welchen der Volumenstrom in einer Schicht mit reflektierender Struktur gemessen werden kann.

Diese Aufgabe wird durch das Verfahren nach dem Wortlaut von Anspruch 1 gelöst bzw. durch die Anordnung gemäss Anspruch 14.

Grundsätzlich werden darüber hinaus die erwähnten Nachteile dadurch behoben, dass gleichzeitig das ganze interessierende, zweidimensionale Gebiet beschallt wird und, abgesehen von Laufzeitdifferenzen, welche durch unterschiedliche räumliche Lagen von Volumenelementen in der interessierenden Schicht relativ zu Sender und/oder Empfänger bedingt sind, eine bis auf Rechenzeiten simultane Auswertung der Echos aus dem gesamten interessierenden Zielgebiet vorgenommen wird.

Obwohl es durchaus möglich ist, die Sende-Wellenpakete so zu erzeugen, dass sie die Schicht nicht kohärent erreichen, um darnach diese Nicht-Kohärenz (Phasenungleichheit) rechnerisch, durch Berücksichtigung von lokal unterschiedlichen Distanzen zwischen Sender und Volumenelementen der Schicht, zu berücksichtigen, werden gemäss Wortlaut von Anspruch 2 in einer bevorzugten Ausführungsvariante die Sende-Wellenpakete so erzeugt, dass sie die Schicht mindestens genähert kohärent (phasengleich) erreichen, womit die Berücksichtigung der genannten unterschiedlichen Sende/Volumenelement-Distanzen entfällt.

Dabei werden die Sende-Wellenpakete bevorzugterweise nach dem Wortlaut von Anspruch 3 erzeugt, womit es durch die zeit- und phasenrichtige Anregung der vorgesehenen Mehrzahl von Quellen möglich wird, gegebenenfalls vorliegende Unterschiede der Distanzen zwischen einzelnen Quellen und einzelnen Volumenelementen der Schicht zu berücksichtigen. Durch das zeit- und phasenrichtige Anregen wird erreicht, dass, wie auch die interessierende Schicht räumlich bezüglich der Mehrzahl vorgesehener Quellen liegen mag, die durch die Mehrzahl von Quellen gesamthaft erzeugten Sende-Wellenpakete, wie bevorzugterweise gefordert, kohärent an der interessierenden Schicht auftreffen.

Einfachere Verhältnisse entstehen, wenn nach dem Wortlaut von Anspruch 4 vorgegangen wird.

Obwohl die Mehrzahl vorgesehener Sensoren durchaus auch in einer dreidimensionalen Anordnung, räumlich, angeordnet werden kann und die aufgrund der Zielschicht und der räumlichen Sensoranordnung entsprechend auftretenden Distanzunterschiede rechnerisch berücksichtigt werden können, wird in einer bevorzugten Ausführungsvariante vorgeschlagen, gemäss Wortlaut von Anspruch 5 vorzugehen.

Da, mit einer im wesentlichen ebenen Senderanordnung, wie auch mit einer im wesentlichen ebenen Sensoranordnung, trotzdem beliebig im Raum liegende Schichten durch rechnerische Berücksichtigung der entsprechenden Sender-Volumenelemente bzw. Volumenelement-Sensoren-Distanzverhältnisse möglich ist, ergibt sich nach dem Wortlaut von Anspruch 6 ein bevorzugtes Vorgehen dadurch, dass Senden und Empfangen von derselben Anordnung aus bewerkstelligt wird und hier eine räumliche, vorzugsweise ebene Anordnung von Sende- und Empfangs-Transducern vorgesehen wird. Damit ergibt sich auch ein kompakter Aufbau von Anregungs- und Auswerte-Elektronik.

Die Sende-Wellenpakete werden, sofern sie mittels einer Mehrzahl von Quellen erzeugt werden, bevorzugterweise nach dem Wortlaut von Anspruch 7 erzeugt. Darnach werden gegebenenfalls notwendige Zeit- und Phasenverschiebungen der Anregungen der einzelnen Quellen mit Bezug auf das erwähnte Referenzsignal definiert.

Im weiteren wird das Referenzsignal, dem Wortlaut von Anspruch 8 folgend, zur Demodulation der Echosignale eingesetzt, wobei vorzugsweise diese Demodulation auf der elektrischen Seite des eigentlich als Schalldruck/elektrischer Wandler ausgebildeten Sensors vorgenommen wird.

Im weiteren werden gemäss Anspruch 9 die Echos, insbesondere die Volumenelementechos, von jedem Sensor digitalisiert und gespeichert, und zwar bevorzugterweise nach der Demodulation der Echosignale.

Durch das weitere bevorzugte Vorgehen gemäss Wortlaut von Anspruch 10 wird erreicht, dass Echos von einem Volumenelement, die auf alle Sensoren auftreffen, rechnerisch gleichphasig gemacht werden, womit je alle Echos von je einem Volumenelement über alle Sensoren gemeinsam ausgewertet werden können.

Grundsätzlich werden gemäss Anspruch 11, durch Bekanntsein der entsprechenden Echo-Empfangszeiten bzw. Distanzen an jedem Sensor, alle Volumenelementechos an jedem Sensor identifiziert und darnach so miteinander verrechnet, dass die an allen Sensoren auftreffenden Echos, jeweils von einem Volumenelement, verstärkt werden, gegenüber den von anderen Volumenelementen an den Sensoren auftreffenden Echos.

Dies wird für alle Volumenelemente durchgeführt, womit letztendlich für jedes Volumenelement von der gesamten Sensoranordnung das Gesamt-Volumenelementechosignal zur Verfügung steht.

Wird, wie oben erläutert wurde, rechnerisch Kohärenz der Echos von einem Volumenelement an allen Sensoren erreicht, so wird die verstärkende, "signal to noise"-verbessernde Verrechnung, vorzugsweise nach dem Wortlaut von Anspruch 12, realisiert.

Diese sensorspezifischen Echos, woraus die Volumenelementechos identifiziert werden, dienen, gemäss Wortlaut von Anspruch 13 gespeichert, als Datenbasis für alle Identifikations- und Verrechnungsschritte zum Erhalt der der Anzahl Volumenelemente entsprechenden Anzahl Gesamt-Volumenelementechos, damit des Schichtechos.

Zum Messen des zweidimensionalen Geschwindigkeitsfeldes der Struktur in der erwähnten Schicht werden ja mindestens zwei Sende-Wellenpakete in erläuterter Art und Weise erzeugt, zeitgestaffelt, und je ihre Echos nach den bisherigen Erläuterungen ausgewertet. Durch Auswerten der Unterschiede, mindestens zweier Sätze von Gesamtvolumenelementechos, wird die Geschwindigkeit der Strukturen in den jeweiligen Volumenelementen ermittelt und, über alle Volumenelemente, schliesslich die Geschwindigkeitsverteilung bzw. das Geschwindigkeitsfeld der Struktur über der Schicht.

Die mit dem erfindungsgemässen Verfahren erwirkten Vorteile bestehen auch darin, dass, für Echtzeitmessungen, das Problem der knappen Messzeit eliminiert wird, indem das ganze interessierende, in die genannten Abtastvolumina aufgeteilte Gebiet simultan erfasst wird. Durch die markant verkürzte Messzeit ist es auch im Echtzeitbetrieb möglich, sowohl eine gute örtliche Auflösung wie auch eine hohe Genauigkeit zu erreichen.

Ein weiterer Vorteil des erfindungsgemässen Verfahrens besteht darin, dass die abgetastete Schicht nicht die Hauptbeschallungsachse enthält bzw. enthalten muss, sondern zu dieser z.B. senkrecht, in wählbarem Abstand von der Sensoranordnung, stehen kann. Dabei ist darauf hinzuweisen, dass durch Vorgabe der Distanzen jedes Sensors von jedem Volumenelement die zweidimensionale Messung unabhängig von der Lage der Schicht zur Hauptbeschallungsachse der Senderanordnung bzw. der Transduceranordnung wird. Mit Hilfe der Distanzvorgabe wird eigentlich festgelegt, welche Schicht ausgemessen werden soll bzw. wie sie im Raume bezüglich der Senderanordnung liegen soll. Dabei versteht es sich von selbst, dass durch Wechseln solcher "Distanzen-Tabellen" eine Schicht- für Schicht-Messung in rascher Abfolge möglich ist.

Es sei auf Moser, "Inhärente Grenzen von Ultraschall-Blutflussmessverfahren", Dissertation, ETH No. 8567, 1988, hingewiesen.

Die Erfindung wird anschliessend beispielsweise anhand von Figuren erläutert.

Es zeigen:

Fig. 1    schematisch die räumliche Anordnung einer bevorzugten Sender/Empfängeranordnung in Form einer Transducermatrix sowie einer auszumessenden Schicht, im Spezialfall eine zur Transducerebene parallele Schicht,

Fig. 2    eine schematische Darstellung der Anregungseinheiten sowie der Echosignal-Auswerteeinheit, zur Signaldigitalisierung und zum Weitermultiplexen der digitalisierten Signale,

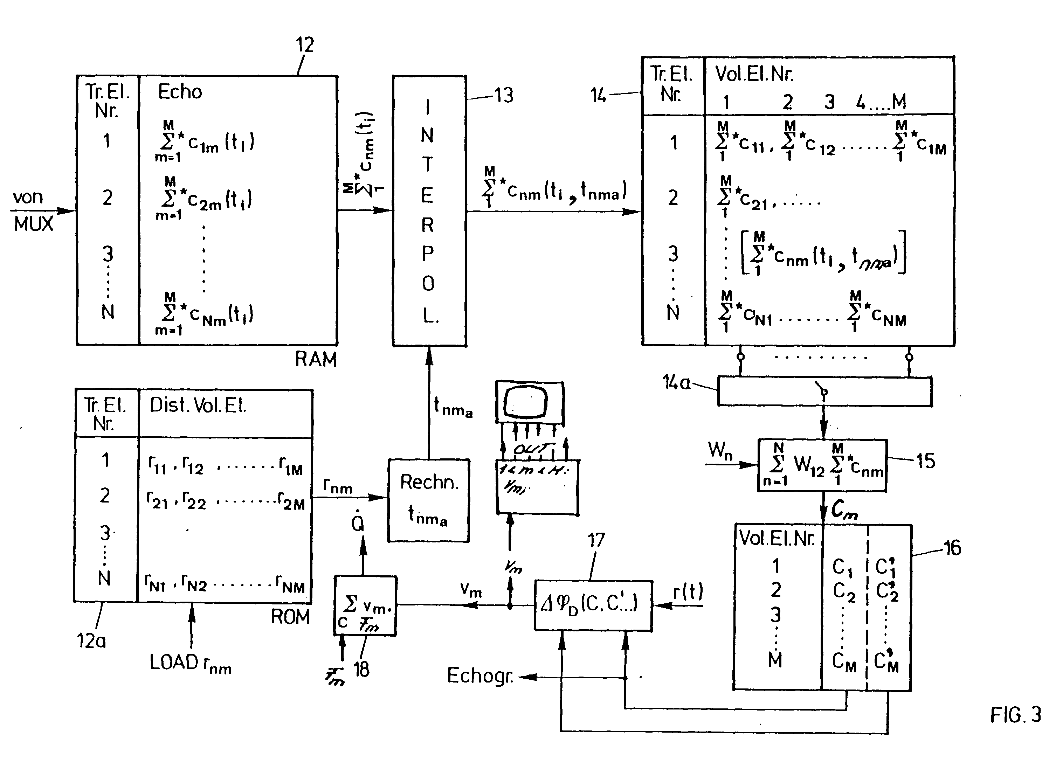

Fig. 3    in Fortsetzung von Fig. 2 die Weiterverarbeitung der Signale ausgangsseitig des Multiplexers gemäss Fig. 2, anhand eines Signalfluss-Funktionsblock-Diagrammes,

Fig. 4a    die Enveloppen-Funktion erfindungsgemäss erzeugter Sende-Wellenpakete über der Zeitachse,

Fig. 4b    das Wellen- bzw. Trägersignal,

Fig. 4c    das mit der Enveloppen-Funktion amplitudenmodulierte Trägersignal gemäss Fig. 4b zur Erzeugung der Sende-Wellenpakete,

Fig. 4d    wiederum über der Zeitachse, ein am Empfangssensorelement von einem Volumenelement aus der Ziel-Schicht erhaltenes Wellenpaket,

Fig. 4e    das Enveloppen-Signal des Echowellenpakets gemäss Fig. 4d, und diesbezüglich die Abtastzeiten $t_i$ gemäss Fig. 2.

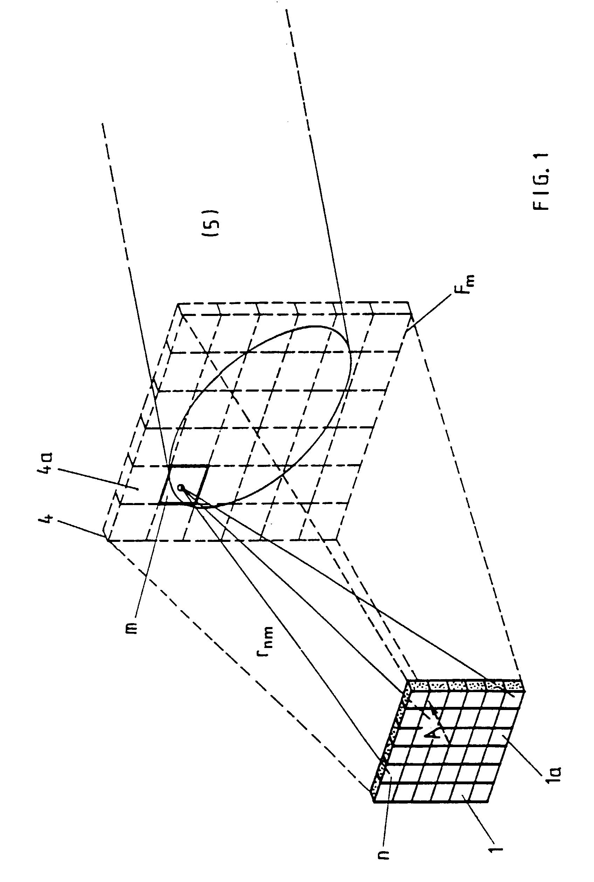

In Fig. 1 ist, schematisch, eine bevorzugte, erfindungsgemässe Anordnung von Quellen zur Erzeugung der Sende-Wellenpakete und von Sensoren zum Empfang der rückgestreuten Echos dargestellt. Bei diesem bevorzugten Ausführungsbeispiel dienen als Sender-Quellen und als Empfangssensoren die gleichen Elemente, sog. Transducer 1, welche in der bevorzugten Ausführungsform im wesentlichen in einer Ebene, matrixförmig, 1a, angeordnet sind.

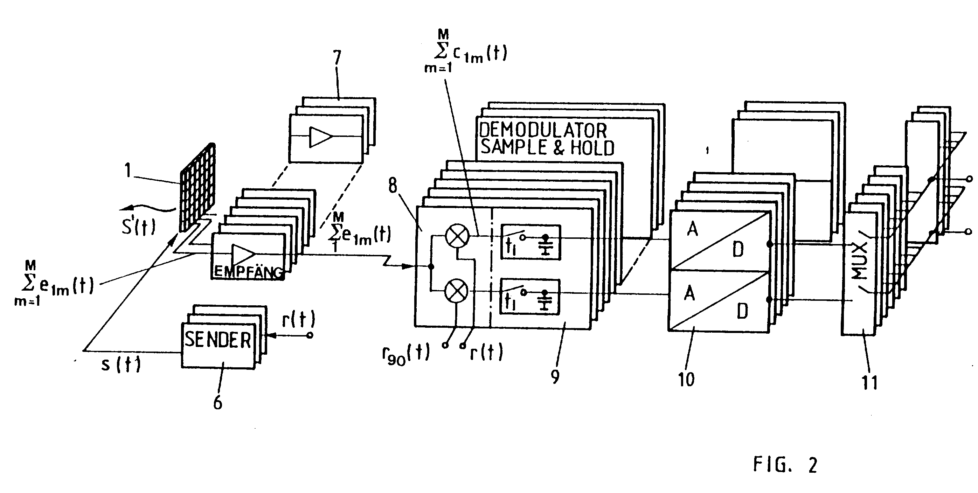

Wie aus Fig. 2 ersichtlich, werden die Transducerelemente 1 mittels eines oder mehrerer Sender 6 zur gemeinsamen Abgabe eines Sende-Wellenpaketes s'(t) angeregt. Wie wiederum aus Fig. 2 ersichtlich, ist den Transducerelementen 1 gemäss Fig. 1 je ein Empfänger 7 zugeschaltet, welcher die vom Transducerelement 1 in elektrische Signale gewandelten Echosignale verstärkt.

Die Ausgangssignale der Empfänger 7 werden, wie in vielkanaligen Doppler-Geräten üblich (siehe "McLeod"), mit Hilfe eines Synchrondemodulators orthogonal ins Basisband gemischt. Hierzu werden zwei orthogonale Referenzsignale r(t) und $r_{90}$(t) dem Synchrondemodulator 8 zugeführt, wobei das Referenzsignal r(t) auch zur Anregung der Transducerelemente 1 über die Sender 6 eingesetzt wird.

Zu wenigen, z.B. vier, vorgegebenen Zeitpunkten $t_i$ werden die am Synchrondemodulator 8 demodulierten orthogonalen Echosignale von jedem Transducerelement 1 an einem Abtast- und Haltekreis 9 abgetastet und je einem A/D-Wandler 10 zugeführt, dort digitalisiert. Die an den A/D-Wandlern 10 digitalisierten Werte gelangen über einen Multiplexer 11 zur Zwischenspeicherung in einen Speicher 12, bevorzugterweise einen RAM-Speicher.

Die Transducerelemente 1 erzeugen Wellenpakete s'(t) - mindestens zwei zur Geschwindigkeitsfelderfassung. Mit der Trägerfrequenz $\omega_o$ kann ein Sende-Wellenpaket wie folgt angeschrieben werden:

$$s'(t) = A(t)e^{j\omega_0 t} \tag{1}$$

Dabei bezeichnen:

A(t):    die Enveloppe des Sende-Wellenpakets

t :    die Zeit

$\omega_o$ :    die Trägerkreisfrequenz

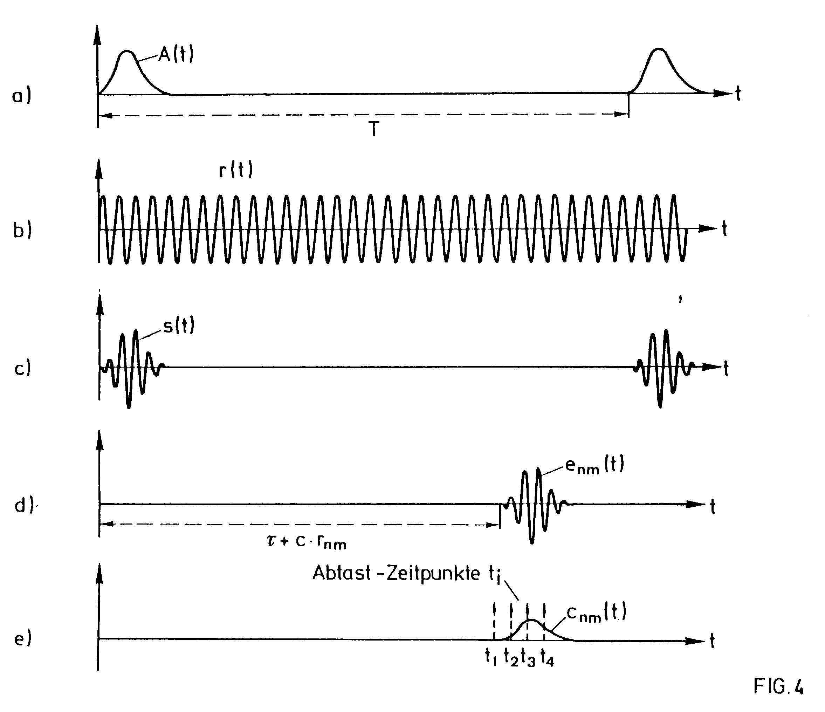

Die Enveloppe A(t) des Sende-Wellenpaketes gemäss Fig. 4a ist, verglichen mit der Repetitionszeit T bei Geschwindigkeitsfeldmessung, nur kurzzeitig verschieden von Null. Das Sende-Wellenpaket s'(t) kann als Produkt der Enveloppe A(t) mit dem quarzstabilen Referenzsignal r(t) angesehen werden, wobei das Referenzsignal r(t) in Fig. 4b, das resultierende Sende-Wellenpaket in Fig. 4c dargestellt sind.

Das Referenzsignal r(t) kann dargestellt werden:

$$r(t) = e^{j\omega_0 t} \tag{2}$$

Das Anregungssignal s(t) gemäss Fig. 2 wird durch die Transducerelemente 1 in die jeweiligen akustischen Sende-Wellenpakete s'(t) gewandelt, welche ins Schallmedium propagieren, wo Anteile davon, durch die im Schallmedium stationär enthaltene oder, in einem Fluidum als Schallmedium, darin bewegte reflektierende Struktur reflektiert werden.

Gemäss Fig. 1 wird nun die Schichtfläche 4, welche auszumessen ist, in eine Anzahl M Volumenelemente unterteilt, wie dargestellt bevorzugterweise gleich grosse, wobei in Fig. 1 der Spezialfall dargestellt ist, in dem die Schicht 4 in einer parallel zur Transducerebene liegenden Ebene liegt.

Es werde nun den gesamthaft M vorgesehenen Volumenelementen die Laufnummer m mit $1 < m < M$ zugeordnet und es sei weiter die Transducermatrix 1a mit gesamthaft N Transducerelementen 1 versehen, welche mit der Laufnummer n mit $1 < n < N$ numeriert seien. Es erzeugt beim Transducerelement No. n das Volumenelement No. m folgendes Echosignal:

$$e_{nm}(t) = D\ A(t-\tau-r_{nm}/c)e^{j\omega_0(t-\tau)-jkT_{nm}} \tag{3}$$

mit

$k=\omega_o/c$:          Wellenzahl

c:          Schallgeschwindigkeit

D:          Verlustraktor

Dabei bedeutet $\tau$ die Laufzeit des Sende-Wellenpaketes von der Transducerebene zur Schicht 4, wobei diese Laufzeit im betrachteten Spezialfall für alle Volumenelemente m gleich gesetzt wird. D berücksichtigt die Dämpfung des Schalls im Medium sowie Streuverluste.

Die Phase des Empfangssignals an einem Transducerelement No. n von einem Volumenelement No. m, d.h. das Echosignal $e_{nm}$ reagiert sehr empfindlich auf Aenderungen der Distanz $r_{nm}$, welche von einem betrachteten Transducerelement 1 zu einem betrachteten Volumenelement definiert ist. (Im allgemeinen Fall sind analog unterschiedliche $\tau_m$ zu berücksichtigen.) Von einem betrachteten Volumenelement No. m empfängt mithin jedes Transducerelement 1 der Matrix 1a ein Echosignal in einer anderen Phasenlage, bedingt durch die unterschiedlichen Distanzen der Volumenelemente 4a zum einen betrachteten Transducerelement 1. Die Amplitude A(t) hingegen ist eine verhältnismässig mit der Distanz wenig variierende Grösse.

Wie in Fig. 2 dargestellt, erscheint am elektrischen Ausgang jedes Transducerelementes 1, beispielsweise desjenigen mit der No. 1, ein Echo, welches sich aus der Gesamtheit des von jedem Volumenelement erzeugten Echos $e_{1m}$ ergibt. Dieses Echosignal jedes Transducerelementes 1 wird am Empfänger 7 verstärkt, und darnach am Synchrondemodulator 8 demoduliert.

In der bevorzugten dargestellten Ausführungsvariante werden nun, wie auch in Fig. 4e dargestellt, zu wenigen vorgegebenen Abtastzeitpunkten $t_i$ die orthogonalen Signale ausgangsseitig des Synchrondemodulators 8 abgetastet, wobei (hier nicht dargestellt) in üblicher Art und Weise vom demodulierten Signal erst Signalanteile mit der Summenfrequenz $2\,\omega_o$ ausgefiltert werden. Ausgangsseitig des Synchrondemodulators erscheint, bezogen auf das beispielsweise besprochene Transducerelement No. 1, die Summe aller komplexen Volumenelement-Echo-Enveloppen, wobei sich, von einem Volumenelement m, diese komplexe Enveloppe, bezogen auf ein Transducerelement n, ergibt gemäss:

$$c_{nm}(t) = D\ A(t-\tau-r_{nm}/c)e^{-j\omega_0\tau-jkr_{nm}} \tag{4}$$

Dieses Signal enthält die volle Information des HF-Signals. Da das Signal bandbegrenzt ist, genügen zur Digitalisierung einige wenige, z.B. vier aequidistant liegende Stützpunkte bei den Abtastzeiten $t_i$, wie in Fig. 4e dargestellt. Die nun zu den erwähnten Zeitpunkten $t_i$ abgetasteten demodulierten Signale werden, wie erwähnt wurde, an den A/D-Wandlern 10 gewandelt und in digitalisierter Form der Multiplexereinheit 11 zugeführt. Ueber die Multiplexereinheit 11 werden sie nun in die Speichereinheit 12 geladen. Im Speicher 12 liegen mithin die digitalisierten Summen der komplexen Enveloppen-Signale, zugeordnet den Transducerelement-No. n=1, 2 ... N.

Es geht nun darum, aus diesem Summensignal - abgetastete (*) Summen der komplexen Enveloppen $c_{nm}$ - zu strukturieren, welcher Echoanteil von welchem Volumenelement an einem betrachteten Transducerelement herrührt.

Nun sind in einem Speicher 12a, beispielsweise einem ROM-, PROM-, EPROM-Speicher, zu jedem Transducerelement n=1 bis N, die Distanzgrössen $r_{nm}$ zu den jeweiligen Volumenelementen 4a in der Schicht 4 gemäss Fig. 1 abgespeichert.

Eigentlich wird durch Festlegung dieser r-Distanzgrössen festgelegt, wie die Schicht 4 gemäss Fig. 1 bezüglich der Transduceranordnung 1a liegen soll.

Aus diesen unterschiedlichen Distanzgrössen $r_{nm}$, womit eigentlich Volumenelement/Transducerelement-Laufzeitspezifische Phasenunterschiede $\varphi_{nm}$ definiert sind, werden "virtuelle" SOLL-Abtastzeiten $t_{nma}$ berechnet, bei denen das jeweilige Transducerelementspezifische Summenechosignal am Ausgang des Synchrondemodulators 8 abgetastet werden müsste, damit, zu diesen vorgegebenen Zeitpunkten abgetastet, die so abgetasteten Signale dem Signalanteil entsprächen, der je von einem betrachteten Volumenelement auf das betrachtete Transducerelement 1 auftrifft.

Nun werden einerseits vom Speicher 12 die jedem Transducerelement zugeordneten Summensignale der komplexen Enveloppen einem Interpolator 13 zugeführt, ebenso wie die ausgehend von den Distanzwerten $r_{nm}$ ermittelten SOLL-Abtastzeiten $t_{nma}$. Im Interpolator werden nun die eigentlich benötigten Werte der komplexen Enveloppensumme durch numerische Interpolation zu den eigentlich erforderlichen Volumenelement-Transducerelement-Abtastzeiten $t_{nma}$ ermittelt.

Es resultieren ausgangsseitig des Interpolators die an jedem Transducerelement der No. n ermittelten, komplexen Enveloppen jedes Volumenelementes m, unter Berücksichtigung der Phasendrehung entsprechend den fiktiven Abtastzeiten bzw. Interpolations-Abtastzeiten $t_{nma}$. Die nun zu den Volumenelement/Sensor-spezifischen Abtastzeiten $t_{nma}$ rechnerisch durch Interpolation ermittelten Werte sind aber weiterhin in dem Sinne "verrauscht", als dass auch bei diesen $t_{nma}$-Abtastwerten noch von anderen Volumenelementen 4a herrührende Anteile vorhanden sind. Die gemäss den $t_{nma}$-Tastzeiten interpolierten Signale werden nun in den Speicher 14 geladen, worin, nun matrixartig, jedem Transducerelement, für jedes Volumenelement die so interpolierte komplexe Enveloppen-Summe aus den Enveloppen:

$$c_{nm}{}^* = c_{nm}(t_i)e^{j\omega_0(t_{nma}-t_i)} = c_{nm}(t_i)e^{j\varphi_{nm}}, \tag{5}$$

i=1,2,3 oder 4 zugeordnet sind.

Dabei bezeichnet:

$\varphi_{nm}$ : eine von der Distanz $r_{nm}$ abhängige Phasendifferenz.

Der Interpolator 13 führt diese komplexe Multiplikation durch, wobei er aus dem Tabellenspeicher 12a die den jeweiligen Volumenelementen bzw. Transducerelementen zugehörigen Werte ausliest.

Die Summen der komplexen Enveloppen im Speicher 14 bestehen je aus Abtastwerten der an jedem Transducerelement empfangenen Gesamtechos, die dann aufgenommen sind, wenn an jedem Transducerelement das Echo von je einem Volumenelement aufgrund der Transducerelement-Volumenelement-Distanz, eintrifft.

Betrachtet man diese Abtastwerte, die wie erwähnt, noch verrauscht sind, für ein Volumenelement an allen Transducerelementen, so entsprechen sie, da die Distanzunterschiede durch die spezifischen $t_{nma}$-Interpolationszeiten berücksichtigt sind, kohärent an allen Transducerelementen ankommenden Echosignalen von jeweils einem Volumenelement, verrauscht durch nichtkohärent angekommene Echosignale der anderen Volumenelemente, für die die jeweiligen $t_{nma}$-Zeitpunkte nicht stimmen. Im Sinne dieser Kohärenz sind somit an allen Transducerelementen die Echos von einem Volumenelement nun korreliert.

Es werden nun alle von einem Volumenelement abgespeicherten komplexen Summen Enveloppenwerte aus den $\Sigma^*c_{nm}$, über alle Transducerelemente, wie mit der Umschaltereinheit 14a schematisch angedeutet, an einem Summator 15 aufsummiert, wobei durch geeignete Wahl der Gewichtungsfaktoren $W_n$ das Uebersprechen zwischen den einzelnen Volumenelementen noch weiter unterdrückt werden kann. Die erwähnte Korreliertheit tritt nun gegenüber der Nicht-Korreliertheit verstärkt hervor. Am Ausgang des Summators 15 treten somit der Reihe nach Summensignale $C_m$ auf, d.h. die über alle Transducerelemente 1 summierten Summen-Echosignale von einem jeweils betrachteten Volumenelement mit der No. m. Diese Summensignale (von Summensignalen mit korreliertem "Summanden") ergeben

sich nach:

$$C_m = \sum_{n=1}^{N} H_n \cdot \sum_{1}^{H} C_{nm}^{*} (t_i - t_{nm_a})$$   (6)

Diese nur mehr Volumenelement-spezifischen Echosignale werden in einem Speicher 16 abgelegt.

Die komplexen Werte $C_m$ enthalten Information über die im Volumenelement enthaltenen reflektierenden Strukturen: Der Betrag ist ein Mass für die Reflexion, die Phase hängt von der relativen Position der reflektierenden Strukturen ab.

Es werden weiter, wie bereits in Fig. 4a dargestellt, mindestens zwei Sende-Wellenpakete s'(t) abgesandt und die Echos beider, durch die lange Zeit T getrennten Pakete, je, wie bis anhin erläutert wurde, ausgewertet.

Es stehen somit durch die mindestens zwei Sende-Wellenpakete und ihre Auswertung im Speicher 16, von jedem Volumenelement mindestens zwei komplexe Werte $C_m$ und $C'_m$ zur Verfügung. Aus Klarheitsgründen und um nicht die ganze Auswertungsanordnung doppelt erläutern zu müssen, sind lediglich im Endspeicher 16 die bei zwei oder mehr Wellenpakten resultierenden, je den Volumenelementen zugeordneten komplexen Werte $C_m$ und $C'_m$ eingetragen.

Aus der Phasenänderung $\Delta\varphi_m(C, C')$, d.h. der Phasenänderung der komplexen Werte $C_m$ von Wellenpaketecho zu Wellenpaketecho wird die Geschwindigkeit $v_m$ der Struktur im Sinne der Doppler-Frequenzauswertung bestimmt, d.h. in Beschallungsrichtung.

Dazu werden, im bevorzugten Fall, bei mehreren Sende-Wellenpaketen, eine zeitliche Folge von Werten $C_m$, $C'_m$, $C''_m$ etc., welche Stützwerte der in den m-ten Volumenelementen generierten Doppler-Frequenzen darstellen, in bekannter Art und Weise analysiert. Ein digitales Hochpassfilter (nicht dargestellt) unterdrückt die stationären Anteile, die z.B. von Gefässwänden herrühren können und im allgemeinen dem dopplerverschobenen Signal überlagert sind. Das gefilterte Signal gelangt sodann zum Frequenzdetektor 17, welcher die mittlere Dopplerfrequenz bestimmt und damit pro Volumenelement die Geschwindigkeit $v_m$ der Struktur.

Durch Summation der für jedes Volumenelement m so ermittelten Doppler-Frequenz $f_{Dm}$ wird der Volumenstrom Q durch die Schicht 4 gemäss Fig. 1 nach folgender Formel bestimmt:

$$\dot{Q} = \frac{c}{2f_0} \sum_{1}^{H} F_m f_{Dm}$$   (7)

$F_m$ :   Fläche des auf die Messebene projizierten Volumenelementes, Messebene senkrecht zur Beschallungsrichtung

c :   Schallgeschwidigkeit

$f_o$ :   Frequenz von r(t)

$f_{Dm}$ :   Dopplerfrequenz im m-ten Volumenelement

Der Gewichtungsfaktor $F_m$ berücksichtigt dabei die Grösse der Teilflächen der Volumenelemente m.

Der Volumenstrom Q, der so bezüglich einer zur Transducermatrixfläche parallelen Schicht ermittelt wird, ist unabhängig von der Richtung der Geschwindigkeitsvektoren durch diese Schicht.

**Patentansprüche**

1.   Verfahren zum Messen des Volumenstromes in einer Schicht (4) mit reflektierender Struktur eines Materialvolumens, mittels Auswertung von Schall- oder Ultraschallecho ($e_{nm}$), dadurch gekennzeichnet, dass

-   mindestens zwei Sende-Wellenpakete (S' (t)) zeitgestaffelt erzeugt werden,

-   eine Mehrzahl Schall- oder Ultraschallsensoren (1) in einer mindestens eindimensionalen Anordnung (1a) vorgesehen wird,

- der Schicht (4) ein Muster von Volumenelementen (4a) zugeordnet wird, wobei mindestens die Distanz ($r_{nm}$) jedes Sensors (1) von jedem Volumenelement (4a) bekannt bzw. vorgegeben ist,

- für jedes Sende-Wellenpaket:

  - für jeden Sensor (1) die Echo-Empfangszeit ($t_{nma}$) von jedem Volumenelement (4a) bestimmt wird,

  - damit aus dem an jedem Sensor (1) empfangenen Echo Volumenelementechos ($\Sigma c^*_{nm}$) selektioniert werden,

  - die Volumenelementechos ($\Sigma c^*_{nm}$) jedes einzelnen Volumenelementes (4a) über alle Sensoren (1) miteinander verrechnet werden, zum Erhalt eines Gesamt-Volumenelementechos ($C_m$) von jedem Volumenelement (4a), das repräsentativ ist für die reflektierende Struktur im einzelnen Volumenelement,

- durch Auswerten der Phase (17) der Gesamt-Volumenelementechowellen ($\Delta\varphi(C_m, C'_m)$) ein zur Geschwindigkeit der Struktur in den Volumenelementen (4a) proportionales Volumenelement-Geschwindigkeitssignal ($v_m$) gebildet wird und

- durch gegebenenfalls gewichtete Summation der Volumenelement-Geschwindigkeitsignale ($v_m$) der Volumenstrom (Q) durch die Schicht bestimmt wird.

2. Verfahren nach Anspruch 1, dadurch gekennzeichnet, dass das Sende-Wellenpaket (s'(t)) so erzeugt wird, dass es die Schicht (4) mindestens genähert kohärent, d.h. mindestens genähert phasengleich, erreicht.

3. Verfahren nach einem der Ansprüche 1 oder 2, dadurch gekennzeichnet, dass die Sende-Wellenpakete (S' (t)) durch zeit- und phasenrichtige Anregung (s(t)) einer Mehrzahl von Schall- oder Ultraschallquellen (1) erzeugt werden.

4. Verfahren nach einem der Ansprüche 1 bis 3, dadurch gekennzeichnet, dass die Sende-Wellenpakete (s'(t)) durch mindestens genähert gleichzeitige Anregung einer Mehrzahl mindestens genähert in einer Ebene (1a) angeordneter Schall- oder Ultraschallquellen (1) erzeugt werden.

5. Verfahren nach einem der Ansprüche 1 bis 4, dadurch gekennzeichnet, dass die Sensoren (1) matrixförmig (1a), mindestens genähert in einer Ebene angeordnet werden.

6. Verfahren nach einem der Ansprüche 1 bis 5, dadurch gekennzeichnet, dass Quellen (1) zur Erzeugung der Sende-Wellenpakete (s'(t)) und die Sensoren (1) mittels einer räumlichen Anordnung von Sende- und Empfangstransducern (1) gebildet werden, vorzugsweise einer mindestens genähert ebenen (1a).

7. Verfahren nach einem der Ansprüche 1 bis 6, dadurch gekennzeichnet, dass die Sende-Wellenpakete (s'(t)) mittels einer Mehrzahl Schall- oder Ultraschallquellen (1) erzeugt werden, die je von einem, von einem stabilen, vorzugsweise quarzstabilen Referenzsignal (r(t)) mindestens abgeleiteten Anregungssignal angeregt werden.

8. Verfahren nach Anspruch 7, dadurch gekennzeichnet, dass das Referenzsignal (r(t)) zur Demodulation des Echos, vorzugsweise zur Demodulation je der von den Sensoren (1) gewandelten Echos eingesetzt wird.

9. Verfahren nach einem der Ansprüche 1 bis 8, dadurch gekennzeichnet, dass die Echos von jedem Sensor (1) digitalisiert (10) und gespeichert (12) werden, vorzugsweise nach deren Demodulation (8).

10. Verfahren nach einem der Ansprüche 1 bis 9, dadurch gekennzeichnet, dass die Echos von jedem Sensor (1) digitalisiert werden, die unterschiedlichen Laufzeiten, entsprechend unterschiedlichen Echo-Empfangszeiten von Volumenelementechos, an allen Sensoren (1) durch entsprechende rechnerische Phasendrehung ($t_{nma}$) kompensiert werden, derart, dass von allen Sensoren (1) gleichphasige, laufzeitkompensierte Volumenelement-Echosignale ($\Sigma c^*_{nm}$) vorliegen.

11. Verfahren nach einem der Ansprüche 1 bis 10, dadurch gekennzeichnet, dass die Verrechnung der Echos aller Sensoren (1) so erfolgt, dass dabei das Echo eines jeweiligen Volumenelementes (m) verstärkt wird, gegenüber Echos von allen anderen Volumenelementen.

**12.** Verfahren nach Anspruch 11, dadurch gekennzeichnet, dass die laufzeitkompensierten Volumenelement-Echosignale ($\Sigma c^*_{nm}$), wie durch Superposition (15) miteinander verrechnet werden und durch diese Korrelations-Verrechnung von anderen Volumenelementen stammende Volumenelementechos unterdrückt werden.

**13.** Verfahren nach Anspruch 9, dadurch gekennzeichnet, dass die gespeicherten (12) Echos je von allen Sensoren (1) als Datenbasis für die Verrechnung zum Erhalt der Gesamt-Volumenelementechos ($C_m$) aller Volumenelemente (4a) eingesetzt werden.

**14.** Messanordnung zum Messen des Volumenstromes ($\dot{Q}$) in einer Schicht (4) mit reflektierender Struktur durch ein Materialvolumen, mittels Auswertung von Schall- oder Ultraschallecho, dadurch gekennzeichnet, dass vorgesehen sind:

- eine Senderanordnung (1, 1a) zur Beschallung des Materialvolumens,

- eine Anordnung räumlich verteilter Empfangssensoren (1),

- jedem Sensor (1) nachgeschaltet:

- eine Digitalisierungs- und eine gemeinsame Korrelationsanordnung (12, 12a, 13, 14, 15) zur Digitalisierung und zur Strukturierung des von den Empfangssensoren empfangenen Echos,

und dass

- der Senderanordnung (1) eine Anregungseinheit (6) zugeschaltet ist, die die Senderanordnung zur Abgabe mindestens von zwei zeitlich getrennten Sende-Wellenpaketen (s'(t)) anregt und

- ein Frequenz- bzw. Phasendetektor (17) vorgesehen ist, um auf der Basis des Doppler-Effektes die Strukturgeschwindigkeit in der Schicht zu berechnen, weiter

- dem Phasen- bzw. Frequenzdetektor (17) ein Summator (18) nachgeschaltet ist, um aus den Geschwindigkeitssignalen den Volumenstrom durch die Schicht zu berechnen.

**15.** Messanordnung nach Anspruch 14, dadurch gekennzeichnet, dass die Korrelationsanordnung einen Summator (15) umfasst, dem alle Empfangssensoren vorgeschaltet sind.

**16.** Messanordnung nach Anspruch 15, dadurch gekennzeichnet, dass den Digitalisierungsanordnungen und dem Summator (15) ein Interpolator (13) zwischengeschaltet ist.

**17.** Messanordnung nach einem der Ansprüche 15 oder 16, dadurch gekennzeichnet, dass dem Summator (15) der Korrelationsanordnung ein Speicher (16) nachgeschaltet ist, worin die Summator-Ausgangsdaten für jedes Sende-Wellenpaket gespeichert werden, und dass der Frequenz- bzw. Phasendetektor (17) dem Speicher (16) nachgeschaltet ist, um aus den Summator-Ausgangsdaten für die Wellenpakete die Strukturgeschwindigkeit in der Schicht (4) zu berechnen.

**Claims**

**1.** A method for measuring the volume flow in a layer (4) with a reflecting structure of a material volume by evaluating a sonic or ultrasonic echo ($e_{nm}$), characterized in that

- at least two emission wave packets (s'(t)) are generated staggered in time,
- a plurality of sound or ultrasound detectors (1) are provided in at least one-dimensional arrangement (1a),
- a pattern of volume elements (4a) is assigned to the layer (4), whereby at least the distance ($r_{nm}$) between each detector (1) and each volume element (4a) is known or predetermined,
- for each emission wave packet :

- the echo reception time ($t_{nma}$) of each volume element (4a) is defined for each detector (1),
- volume element echoes ($\Sigma c^*_{nm}$) are selected from the echo received at each detector (1),

- the volume element echoes ($\Sigma c^*_{nm}$) of each individual volume element (4a) are compared to each other over all detectors (1) in order to obtain a total volume element echo ($C_m$) of each volume element (4a) which is representative of the reflecting structure in the individual volume element,

- by evaluating the phase (17) of the total volume element echo waves ($\Delta\varphi(C_m, C'_m)$), a volume element speed signal ($v_m$) proportional to the speed of the structure in the volume elements (4a) is formed and
- the volume flow ($\dot{Q}$) through the layer is defined by means of a possibly weighted summing of the volume element speed signals ($V_m$).

2. The method according to claim 1, characterized in that the emission wave packet (s'(t)) is generated so that it reaches the layer (4) at least approximately in a coherent way, i.e. at least approximately in phase.

3. The method according to claim 1 or 2, characterized in that the emission wave packets (s'(t)) are generated by an excitation (s(t)) with exact time and phase of a plurality of sonic or ultrasonic sources (1).

4. The method according to one of claims 1 to 3, characterized in that the emission wave packets (s'(t)) are generated by at least approximately simultaneously exciting a plurality of sonic or ultrasonic sources (1) arranged at least approximately in one plane (1a).

5. The method according to one of claims 1 to 4, characterized in that the detectors (1) are arranged in a matrix form (1a), at least approximately in one plane.

6. The method according to one of claims 1 to 5, characterized in that sources (1) for generating the emission wave packets (s'(t)) and the detectors (1) are formed by means of a spatial arrangement of emission and reception transducers (1) which is preferably at least approximately plane (1a).

7. The method according to one of claims 1 to 6, characterized in that the emission wave packets (s'(t)) are generated by means of a plurality of sonic or ultrasonic sources (1) which are excited by a respective excitation signal at least derived from a stable, preferably crystalstabilized reference signal (r(t)).

8. The method according to claim 7, characterized in that the reference signal (r(t)) is used for demodulating the echo and preferably for demodulating the echo converted by the detectors (1).

9. The method according to one of claims 1 to 8, characterized in that the echoes are digitalized (10) and stored (12) by each detector (1), preferably after having been demodulated (8).

10. The method according to one of claims 1 to 9, characterized in that the echoes are digitalized by each detector (1) and the different propagation times corresponding to the different reception times of the volume element echoes are compensated on all detectors (1) by a corresponding calculated phase angle rotation ($t_{nma}$) so that on all detectors (1) there are in-phase volume element echo signals ($\Sigma c^*_{nm}$) with compensated propagation time.

11. The method according to one of claims 1 to 10, characterized in that the comparison of the echoes of all detectors (1) takes place so that the echo of a volume element (m) is amplified compared to the echoes of all the other volume elements.

12. The method according to claim 11, characterized in that the volume element echo signals ($\Sigma c^*_{nm}$) with compensated propagation time are compared to each other by superposition (15) for example and through this correlation comparison the volume element echoes coming from other volume elements are suppressed.

13. The method according to claim 9, characterized in that the stored (12) echoes are used by all detectors (1) as a data base for the comparison to obtain the total volume element echoes ($C_m$) of all volume elements (4a).

14. A measuring device for measuring the volume flow ($\dot{Q}$) in a layer (4) with a reflecting structure through a material volume by evaluating a sonic or ultrasonic echo ($e_{nm}$), characterized in that there are provided :

- a transmitting device (1, 1a) for exposing the material volume to sound waves,
- an arrangement of reception detectors (1) distributed in space,
- connected after each detector (1) :

- a digitalizing and a common correlation device (12, 12a, 13, 14, 15) for digitalizing and structuring the echoe received by the reception detectors,

and in that

- an excitation unit (6) is connected to the transmitting device (1), said excitation unit (6) exciting the latter in order to deliver at least two emission wave packets (s'(t)) separate in time,
- there is provided a frequence or phase detector (17) for calculating the structure speed in the layer on the basis of Doppler's effect, and
- a summing integrator (18) is connected after phase or frequence detector (17) to calculate the volume flow through the layer from the speed signals.

15. The measuring device according to claim 14, characterized in that the correlating device includes a summing integrator (15) before which all reception detectors are connected.

16. The measuring device according to claim 15, characterized in that an interpolator (13) is inserted between the digitalizing devices and the summing integrator (15).

17. The measuring device according to claim 15 or 16, characterized in that there is provided, connected after the summing integrator (15) of the correlating device, a memory (16) where the summing integrator's output data are stored for each emission wave packet, and in that frequence of phase detector (17) is connected after memory (16) in order to calculate the structure speed in the layer (4) from said summing integrator output data for the wave packets.

**Revendications**

1. Procédé pour mesurer le courant de volume dans une couche (4) à structure réfléchissante d'un volume de matériau grâce à l'évaluation d'un écho de son ou d'ultrason ($e_{nm}$), caractérisé

   - en ce qu'au moins deux paquets d'ondes d'émission (s'(t)) sont générés de façon échelonnée dans le temps,
   - en ce qu'il est prévu une multiplicité de capteurs acoustiques ou ultrasonores (1) suivant une disposition au moins unidimensionnelle (1a),
   - en ce qu'on associe à la couche (4) un modèle d'éléments de volume (4a), étant précisé qu'on connaît ou qu'on prédéfinit au moins la distance ($r_{nm}$) entre chaque capteur (1) et chaque élément de volume (4a),
   - en ce que pour chaque paquet d'ondes d'émission,

     - on définit pour chaque capteur (1) le temps de réception d'écho ($t_{nma}$) de chaque élément de volume (4a),
     - on sélectionne ainsi à partir de l'écho reçu au niveau de chaque capteur (1) des échos d'éléments de volume ($\Sigma c^*_{nm}$),
     - on compare les échos ($\Sigma c^*_{nm}$) de chaque élément de volume individuel (4a) entre eux sur tous les capteurs (1) afin d'obtenir un écho d'élément de volume global ($c_m$) de chaque élément de volume (4a) qui soit représentatif de la structure réfléchissante dans l'élément de volume individuel,

   - en ce que grâce à l'évaluation de la phase (17) des ondes d'échos d'éléments de volume globaux ($\Delta\varphi(C_m, C'_m)$), on définit un signal de vitesse d'élément de volume ($v_m$) proportionnel à la vitesse de la structure dans les éléments de volume (4a), et
   - en ce que grâce à une somme éventuellement pondérée des signaux de vitesse d'élément de volume ($v_m$), on définit le courant de volume (Q) à travers la couche.

2. Procédé selon la revendication 1, caractérisé en ce que le paquet d'ondes d'émission (s'(t)) est généré de manière à atteindre la couche (4) d'une manière au moins approximativement cohérente, c'est-à-dire au moins approximativement en phase.

3. Procédé selon la revendication 1 ou 2, caractérisé en ce que les paquets d'ondes d'émission (s'(t)) sont générés par l'excitation (s(t)) de durée et de phase correctes d'une multiplicité de sources sonores ou ultrasonores (1).

4. Procédé selon l'une des revendications 1 à 3, caractérisé en ce que les paquets d'ondes d'émission (s'(t)) sont

EP 0 616 231 B1

générés par une excitation au moins approximativement simultanée d'une multiplicité de sources sonores ou ultrasonores (1) disposées au moins approximativement dans un plan (1a).

5. Procédé selon l'une des revendications 1 à 4, caractérisée en ce que les capteurs (1) sont disposés en forme de matrice (1a) au moins approximativement dans un plan.

6. Procédé selon l'une des revendications 1 à 5, caractérisé en ce que des sources (1) pour générer les paquets d'ondes d'émission (s'(t)) et les capteurs (1) sont formés grâce à une disposition dans l'espace de transducteurs d'émission et de réception (1), de préférence au moins approximativement plane (1a) .

7. Procédé selon l'une des revendications 1 à 6, caractérisé en ce que les paquets d'ondes d'émission (s'(t)) sont générés à l'aide d'une multiplicité de sources sonores ou ultrasonores (1) qui sont excitées chacune par un signal d'excitation au moins dérivé d'un signal de référence (r(t)) stable, de préférence stabilisé par cristal.

8. Procédé selon la revendication 7, caractérisé en ce que le signal de référence (r(t)) sert à la démodulation de l'écho, de préférence à la démodulation des échos transformés par les capteurs (1).

9. Procédé selon l'une des revendications 1 à 8, caractérisé en ce que les échos sont numérisés (10) et mis en mémoire (12) par chaque capteur (1), de préférence après leur démodulation (8).

10. Procédé selon l'une des revendications 1 à 9, caractérisé en ce que les échos sont numérisés par chaque capteur (1) et les différentes durées de propagation correspondant aux différents temps de réception des échos d'éléments de volume sont compensées au niveau de tous les capteurs (1) par une rotation de phase numérique ($t_{nma}$) correspondante, de sorte qu'il y a au niveau de tous les capteurs (1) des signaux d'écho d'éléments de volume ($\Sigma c^{*}_{nm}$) de même phase et à durée de propagation compensée.

11. Procédé selon l'une des revendications 1 à 10, caractérisé en ce que la comparaison des échos de tous les capteurs (1) se fait de telle sorte que l'écho d'un élément de volume (m) est amplifié par rapport aux échos de tous les autres éléments de volume.

12. Procédé selon la revendication 11, caractérisé en ce que les signaux d'écho d'éléments de volume ($\Sigma c^{*}_{nm}$) à durées de propagation compensées sont comparés entre eux par exemple par superposition (15) et cette comparaison par corrélation supprime l'écho d'éléments de volume provenant d'autres éléments de volume.

13. Procédé selon la revendication 9, caractérisé en ce que les échos mis en mémoire (12) sont utilisés par tous les capteurs (1) comme base de données pour la comparaison, en vue d'obtenir les échos d'éléments de volume globaux ($C_m$) de tous les éléments de volume (4a).

14. Dispositif de mesure pour mesurer le courant de volume ($\dot{Q}$) dans une couche (4) à structure réfléchissante à l'aide d'un volume de matériau, grâce à l'évaluation d'un écho de son ou d'ultrason, caractérisé en ce qu'il est prévu :

   - un dispositif émetteur (1, la) pour exposer le volume de matériau à l'action de sons,
   - un agencement de capteurs de réception (1) répartis dans l'espace,
   - en aval de chaque capteur (1) :

      - un dispositif de numérisation et un dispositif commun de corrélation (12, 12a, 13, 14, 15) pour numériser et pour structurer l'écho reçu par les capteurs de réception,

   et en ce que

      - une unité d'excitation (6) est associée au dispositif émetteur (1), laquelle unité d'excitation (6) excite celui-ci en vue de l'émission d'au moins deux paquets d'ondes d'émission (s'(t)) séparés dans le temps,
      - il est prévu un détecteur de fréquence ou de phase (17) pour calculer sur la base de l'effet Doppler la vitesse de structure dans la couche, et
      - un sommateur (18) est monté en aval du détecteur de phase ou de fréquence (17) pour calculer à partir des signaux de vitesse le courant de volume à travers la couche.

15. Dispositif de mesure selon la revendication 14, caractérisé en ce que le dispositif de corrélation comprend un sommateur (15) en amont duquel sont montés tous les capteurs de réception.

16. Dispositif de mesure selon la revendication 15, caractérisé en ce qu'un interpolateur (13) est intercalé entre les dispositifs de numérisation et le sommateur (15).

17. Dispositif de mesure selon la revendication 15 ou 16, caractérisé en ce qu'il est prévu, montée en aval du sommateur (15) du dispositif de corrélation, une mémoire (16) dans laquelle sont stockées les données de sortie du sommateur pour chaque paquet d'ondes d'émission, et en ce que le détecteur de fréquence ou de phase (17) est monté en aval de la mémoire (16) pour calculer à partir des données de sortie du sommateur pour les paquets d'ondes la vitesse de structure dans la couche (4).

FIG. 1

EP 0 616 231 B1

FIG. 2

$\sum\limits_{m=1}^{M} c_{1m}(t)$

DEMODULATOR
SAMPLE & HOLD

$\sum\limits_{m=1}^{M} e_{1m}(t)$

$\sum\limits_{1}^{M} e_{1m}(t)$

$S'(t)$

EMPFÄNG

SENDER

$r(t)$

$s(t)$

$r_{90}(t)$    $r(t)$

A  D

A  D

MUX

EP 0 616 231 B1

15

FIG. 3

EP 0 616 231 B1

a) $A(t)$, $T$

b) $r(t)$

c) $s(t)$

d) $e_{nm}(t)$, $\tau + c \cdot r_{nm}$

e) Abtast–Zeitpunkte $t_i$, $c_{nm}(t)$, $t_1\, t_2 t_3\, t_4$

FIG. 4